(19) Europäisches Patentamt
European Patent Office
Office européen des brevets

(11) **EP 1 491 879 A2**

(12) **EUROPÄISCHE PATENTANMELDUNG**

(43) Veröffentlichungstag:
**29.12.2004 Patentblatt 2004/53**

(51) Int Cl.⁷: **G01N 21/88**, G01N 33/46

(21) Anmeldenummer: **04013428.0**

(22) Anmeldetag: **08.06.2004**

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(30) Priorität: **10.06.2003 DE 10326035**

(71) Anmelder: **hema electronic GmbH**
**73431 Aalen (DE)**

(72) Erfinder: **Louban, Roman, Dr.-Ing.**
**74564 Crailsheim (DE)**

(54) **Verfahren zur adaptiven Fehlererkennung auf einer strukturierten Oberfläche**

(57) Die Erfindung betrifft ein Verfahren zur adaptiven Erkennung eines Fehlers *8* auf einer strukturierten Oberfläche *2*, z.B. einer Holzoberfläche, der auf einem mit einem Kamerasensor aufgenommenen Licht-und-Schatten-Bild mit Hilfe konturverfolgungsbasierter Segmentierung (Blobanalyse) gesucht wird. Die dabei genutzte Schwellwerte — die maximale Fehlerhelligkeit $I_{bot}$ *(bottom)* und die minimale Oberflächenhelligkeit $I_{top}$ *(top)*, die dazu dienen den Startpunkt *9* und die weitere Kontur des gesuchten Fehlers *8* (Blob) zu detektieren, - werden angepasst zu lokalen Helligkeitsbedingungen dynamisch aus dem Histogramm des Testbereiches *4* zusammen mit dem Grauwertprofil entlang einer Suchlinie *5* im Testbereich *4* ermittelt, wobei das Histogramm und das Grauwertprofil als Gaußverteilungen behandelt werden.

**Fig. 1**

**Beschreibung**

Die Erfindung betrifft ein Verfahren gemäß dem Oberbegriff des Anspruchs 1.

Insbesondere bezieht sich die Erfindung auf die Erkennung eines Fehlers (Beschädigung) auf einer strukturierten Oberfläche, z.B. einer Holzoberfläche, die als Licht-und-Schatten-Bild mittels eines Bildverarbeitungssystems gewonnen wird. Unabhängig von der Strukturierung der Oberfläche und von dem aufgetretenen Helligkeitsabweichungen gewährleistet das Verfahren eine einwandfreie Erkennung aller vorhandenen Fehler, wobei alle dafür benötigten Parameter angepasst zu lokalen Helligkeitsbedingungen aus dem zu untersuchenden Bild dynamisch ermittelt werden.

Es ist Stand der Technik, dass man für die Erkennung eines Fehlers auf einem zu untersuchendem Bild die konturverfolgungsbasierte Segmentierung (Blobanalyse) (Handbuch der Operatoren für die Bildverarbeitung / Reinhard Klette, Piero Zamperoni. - Braunschweig / Wiesbaden: Friedr. Vieweg & Sohn, 1995) benutzt. Diese ist in einem Testbereich durchzuführen, wobei die Startposition des Fehlers entlang einer Suchlinie, die über alle Zeilen des Testbereiches gelegr werden soll, gesucht wird. Dabei benutzt man die minimale Oberflächenhelligkeit $I_{top}$ (top) und die maximale Fehlerhelligkeit $I_{bot}$ (bottom).

Falls an einer beliebigen x-Stelle gilt:

$$I(x) \leq I_{bot} \, ;$$

$$I(x\text{ -}1) \geq I_{top},$$

wobei: $I(x)$ - die Helligkeit des zu untersuchenden $x$-Pixels entlang der Suchlinie;

$I(x$ - 1) - die Helligkeit des vorherigen $x$-1-Pixels entlang der Suchlinie,

ist die Startposition des Fehlers gefunden worden. Ab dieser Stelle kann der Fehlerkontur weiter mit Hilfe bekannter konventioneller Konturverfolgungsalgorithmen ermittelt werden. Beispielsweise kann dieser nach dem Wert $I_{top}$ durchgeführt werden, wobei alle Pixel, die zum Fehler gehören, eine Helligkeit aufweisen sollen, die unter der minimalen Oberflächenhelligkeit $I_{top}$ liegt.

Dieses Verfahren bezieht sich jedoch auf festen Schwellwerte $I_{bot}$ und $I_{top}$, die auf einer strukturierten und damit inhomogenen Oberfläche keine sichere Fehlererkennung gewährleisten können.

Eine vereinfachte Version dieses Verfahrens (Industrielle Bildverarbeitung / Christian Demant, Bernd Streicher-Abel, Peter Waszkewitz. - Berlin; Heidelberg: Springer, 1998), die einen gleichen Schwellwert $I_{thd}$ (threshold) sowohl für die minimale Oberflächenhelligkeit $I_{top}$ als auch für die maximale Fehlerhelligkeit $I_{bot}$ vorsieht:

$$I_{thd} = I_{bot} = I_{top},$$

was eine Binarisierung des zu untersuchenden Bildes bedeutet, ist noch weniger dafür geeignet.

Seit den achtziger Jahren wird versucht das Problem der Erkennung eines Fehlers auf einer strukturierten Oberfläche mit statistischen Methoden, basierend auf lokalen Texturmerkmalen, zu lösen (Frei konfigurierbare regionalbasierte Farbtexturanalyse zur automatischen Auswertung von Bild-Stichproben / A. Burmeister. - Arbeit zur Erlangung des Grades "Doktor-Ingenieur" - Der Technischen Fakultät der Universität Erlangen-Nürnberg: Erlangen, 1999).

Diese Verfahren liefern aber keine befriedigenden Resultate, obwohl über etwa anderthalbtausend Texturmerkmale zur Fehlererkennung verwendet werden können (Automatische Extraktion von Festigkeits und optisch relevanten Merkmalen aus Farbbildern von Schnittholzflächen. - DGfH-Nr. F-98/01, Projektleitung: Prof. Dr.-Ing. Habil. S. Fuchs, Universität Dresden, Fakultät Informatik, Institut für künstliche Intelligenz, 2002).

Die Schwierigkeiten dieser Aufgabe resultieren aus der hohen Variabilität der einzelnen Fehlerklassen, die dabei von den zulässigen Elementen der Oberflächenstruktur unterschieden werden sollen.

Deswegen kann diese Aufgabe nur dann eindeutig gelöst werden, falls ein Fehler zuerst von seiner Umgebung abgetrennt und danach analysiert wird. Damit soll wieder die konturverfolgungsbasierte Segmentierung verwendet werden. Allerdings soll diese Segmentierung in Form einer Auswertung der fehlerspezifischen und kantencharakteristischen Merkmale durchgeführt werden, die angepasst zu lokalen Helligkeitsbedingungen aus dem zu untersuchenden Bild dynamisch ermittelt werden. Dabei spielt die Kantenerkennung eines Objektes eine zentrale Rolle.

Es ist Stand der Technik, dass man für die generelle Kantensuche eine Umwandlung des gesamten Bildes in ein Kantenbild mit Hilfe verschiedener Kantenfilter (Digitale Bildverarbeitung / Bernd Jähne. - 4. Aufl. - Berlin, Heidelberg, etc.: Springer, 1997) benutzt.

Neben dem erheblichen Rechenaufwand hat diese Methode den Nachteil, dass das Bild durch eine Filterung bearbeitet, d.h. geändert wird, was die Kantenerkennung selbst beeinflusst und damit das Ergebnis verfälscht. Dabei können manche Kanten wegen mangelhaften Kontrasts überhaupt nicht erkannt bzw. zufällige Stellen, die einen ausreichenden Beleuchtungsgradient aufweisen, fälschlicherweise als Kanten erkannt werden.

Ein weiteres Verfahren (DE 19902401 C2) sieht zuerst eine Binarisierung des Bildes vor. Nach der Binarisierung wird eine Kantendetektion vorgenommen. Um eine Binarisierung durchzuführen, muss ein Schwellwert gebildet werden. Dieser kann entweder vorgegeben oder aus dem Bildinhalt dynamisch ermittelt werden.

Der vorgegebene Schwellwert (DE 4131778 C2) berücksichtigt aber keine Abweichungen, z.B. Beleuchtungsschwankungen, die auf einer Serie von aufeinander folgenden Aufnahmen bzw. auf unterschiedlichen Bereichen des aktuellen Bildes vorhanden sind. Dabei kann das zu untersuchende Bild nicht sauber binarisiert werden, was ebenso eine falsche Erkennung der Kanten verursacht.

Den Schwellwert direkt aus dem Inhalt des aktuellen Bildes zu ermitteln, ist eine Möglichkeit das Kantendetektierungsverfahren an das zu untersuchende Bild anzupassen. Dafür benutzt man z.B. ein Histogramm (EP 0750272 B1, EP 0807297 B1), das die Häufigkeit von einzelnen, im Bild auftretenden Grauwerten darstellt. Aus dem absoluten oder lokalen Maximum bzw. Minimum des Histogramms kann ein Binarisierungsschwellwert entnommen werden.

Wenn das Histogramm jedoch für große Bildbereiche bestimmt wird, neigen Kanten dazu, verschmiert oder sogar zerstört zu werden. Wenn umgekehrt der zu binarisierende Bereich zu klein ist, kann dieser Bereich nicht sauber binarisiert werden. Das Verfahren, den zu binarisierenden Bildbereich in geeignete Teilstücke zu unterteilen (EP 0750272 B1), ist wiederum auf vorgegebene Parameter angewiesen. Damit verliert das Verfahren seine Flexibilität.

Eine Reihe von Kandidatenbinärbilder aufzunehmen und auszuwerten, welche mit fallenden bzw. mit steigenden Schwellwerten erzeugt wurden, um den bestmöglichen Binarisierungsschwellwert zu finden (EP 0807297 B1), ist sehr aufwändig und zeitraubend und ist vor allem nur in einer sehr beschränkten Anzahl der Fälle möglich.

Darüber hinaus beeinflusst die Binarisierung des Bildes die Kantenerkennung und damit verfälscht sie, genauso wie jede andere Bildfilterung, das Ergebnis der Kantendetektierung. Deswegen soll die Kantenerkennung am besten auf dem Originalgrauwertbild erfolgen.

Es ist ferner bekannt, dass man eine Kante aus einem Grauwertbild direkt mittels eines Kantenmodells ermitteln kann (Industrielle Bildverarbeitung / Christian Demant, Bernd Streicher-Abel, Peter Waszkewitz. - Berlin; Heidelberg: Springer, 1998). Das betrifft besonders den Fall, wenn der Ort der Kante bereits bekannt ist. Die Suche nach Kanten mit einem Kantenmodell erfolgt normalerweise auf Suchlinien in einer bestimmten Richtung.

Die Kriterien für die Detektierung einer Kante ergeben sich aus dem Grauwertprofil entlang dieser Suchlinie.

Dabei unterscheidet man zwei Kantenrichtungen: ansteigende und abfallende Kanten. Von einer abfallenden Kante spricht man, wenn das Grauwertprofil von hell nach dunkel verläuft, im umgekehrten Fall hat man eine ansteigende Kante vor sich.

Ein typisches Verfahren benutzt folgende Parameter:

- Kantenhöhe: Zur Detektierung einer gültigen Kante fordert man, dass eine bestimmte Mindestdifferenz der Grauwerte entlang der Suchlinie auftritt. Dies bezeichnet man als Kantenhöhe.
- Kantenlänge: Die Kantenlänge gibt an, auf welcher Strecke die durch die Kantenhöhe festgestellte Mindestdifferenz der Grauwerte auftreten muss.

An diesem Verfahren, das als Stand der Technik gilt, ist nachteilig, dass die vorgegebene Kantenhöhe und Kantenlänge bei jedem zu untersuchenden Bild unverändert bleiben müssen, und damit keine dynamische Anpassung an das aktuelle Bild angeboten wird.

Eine andere Lösung ist vom DE 4435796 C1 bekannt, in dem ein Verfahren zur automatischen Erkennung von Kanten und Durchmessern in Bildern beschrieben ist. Mit dessen Hilfe kann eine Kante nach einem vorgegebenen Profil und nach einem vorgegebenen prozentualen Schwellwert, der eine Ähnlichkeitsquote, z.B. mehr als 75%, erreicht hat, ermittelt werden.

Dabei benötigt man aber eine Musterkante, deren Profil weiter benutzt werden soll, und ebenso einen prozentualen Schwellwert als vorgegebenen Parameter. Deswegen bleibt dieses Verfahren vollständig objektspezifisch und bietet keine Anpassung an das zu untersuchende Bild.

Zusammenfassend können die obengenannten Methoden keine flexible und gleichzeitig explizite Kantenerkennung auf einem zu untersuchenden Bild gewährleisten und deswegen auch nicht für die Fehlererkennung auf einer strukturierten Oberfläche benutzt werden.

Der Erfindung liegt die Aufgabe zugrunde, ein Verfahren zur adaptiven Fehlererkennung auf einer strukturierten Oberfläche zu schaffen, das alle dazu benötigten Merkmale angepasst zu lokalen Helligkeitsbedingungen aus dem zu untersuchenden Bild dynamisch ermittelt.

Die Lösung des technischen Problems ergibt sich durch die Merkmale der Patentansprüche 1 bis 14.

Erfindungsgemäß wird die Erkennung eines Fehlers auf einem Bild in einem Testbereich durchgeführt. Aus dem gesamten Testbereich wird das Histogramm und entlang einer Suchlinie, die beispielsweise durch alle Zeilen des Testbereiches gelegt werden soll, das Grauwertprofil gewonnen. Dabei geht man davon aus, dass ein Fehler genauso wie der Hintergrund, beispielsweise immer dunkler als die Oberfläche ist. Ansonsten sollen die Rollen der Oberfläche und des Fehlers vertauscht werden.

Desweiteren muss das Grauwertprofil entlang einer Suchlinie, die quer zur einen typischen Kante liegt, die zwischen der Oberfläche und dem Hintergrund liegt, gewonnen werden.

*Gemäß Anspruch 1* sollen alle Grauwertprofile und das Histogramm als Gaußverteilungen behandelt und gemeinsam als Ausgangsdaten für die Kantendetektierung und damit für die Fehlererkennung verwendet werden. Aus dem zu untersuchenden Bild gewonnenen Histogramm wird zuerst die Oberflächenhelligkeit $I_{surf}$ (*surface*) ermittelt. Desweiteren soll die Standardabweichung $\sigma$ des Grauwertprofils ermittelt, das quer zu einer typischen Kante gewonnen wurde. Diese Parameter sollen weiter genutzt werden, um die maximale Fehlerhelligkeit $I_{bot}$ (*bottom*) und die minimale Oberflächenhelligkeit $I_{top}$ (*top*) für die Detektierung des Startpunktes des gesuchten Fehlers (Blob) dynamisch zu berechnen.

Es ist bekannt, dass die Intensitätsverteilung eines Strahles ein Gaußsches Profil aufweist (Technische Optik: Grundlagen und Anwendungen / Gottfried Schröder. - 7.Aufl. -Würzburg: Vogel, 1990 (Kamprath-Reihe), S. 88). Da eine Oberfläche wegen ihrer Reflexion als Strahlquelle betrachtet werden kann, darf eine Helligkeitsverteilung, die von der Oberfläche über die Kante zum Hintergrund geht, mit einer Gaußverteilung beschrieben werden (Digitale Bildverarbeitung / Bernd Jähne. - 4. Aufl. - Berlin, Heidelberg, etc.: Springer, 1997). Dieses Modell hat sich auch als das Beste für die genaue Berechnung der Kantenposition mit Subpixel-Genauigkeit erwiesen (A Comparison of Algorithms for Sub-pixel Peak Detection / D. K. Naidu, R. B. Fisher. Department of Artifical Intelligence, University of Edinburgh, Scotland, U.K., DAI Research Paper No. 553, Oktober 17, 1991). Deshalb kann für die Beschreibung des Grauwertprofils ein Gaußsches Profil angenommen werden.

Ein Histogramm ist eine Häufigkeitsverteilung der Helligkeit auf einer Oberfläche bzw. ihrem Hintergrund. In natürlichen Bildern hat der Bildinhalt meistens ein mit der Ortsfrequenz abfallendes Amplitudenspektrum, wogegen das Rauschen ein im Frequenzbereich etwa konstantes Spektrum aufweist. Daraus folgt, dass das Histogramm sowie das Grauwertprofil auch ein Gaußsches Profil (Methoden der digitalen Bildsignalverarbeitung / Piero Zamperoni. - Braunschweig / Wiesbaden: Friedr. Vieweg & Sohn, 1989) bzw. ein aus mehreren Gaußverteilungen resultierendes Profil (Digitale Bildverarbeitung / Bernd Jähne. - 4. Aufl. - Berlin, Heidelberg, etc.: Springer, 1997) aufweisen.

Der Helligkeitswert, der am häufigsten an einer Oberfläche aufgetreten ist, kann als Helligkeit dieser Oberfläche definiert werden. Dieses Verfahren weist der zu untersuchenden Oberfläche eine objektive Helligkeit $I_{surf}$ zu, solange eine Störung mit einem bestimmten Helligkeitswert nicht eine größere oder vergleichbare Häufigkeit aufweist, wie die der Oberflächenhelligkeit $I_{surf}$. Dann ist es nicht mehr möglich zwischen der Störung und der Hauptoberfläche selbst zu unterscheiden (z.B. Schachbrett).

Aus dem Histogramm des Testbereiches soll die Oberflächenhelligkeit $I_{surf}$ (*surface*) als der am häufigsten vorkommende Wert ermittelt werden.

Aus dem Grauwertprofil, das an der typischen Kante gewonnen wurde, kann mit Hilfe der bekannten konventioneller Rechenmethoden, z.B. die Methode der kleinsten Quadrate, die Standardabweichung $\sigma$ des Gaußschen Profils ermittelt werden. Diese Standardabweichung $\sigma$ ist eine eindeutige Charakteristik der Kante, und kann erfindungsgemäß als Sollwert der Standardabweichung des Grauwertprofils entlang jeder Suchlinie angenommen werden, die quer zu einer Kante, u.a. an einem Fehler, liegt.

Auf der Basis der gewonnenen Merkmale kann die maximale Fehlerhelligkeit $I_{bot}$, die die größte zulässige Helligkeit eines Fehlers darstellt, sowie die minimale Oberflächenhelligkeit $I_{top}$, die die kleinste zulässige Helligkeit der Oberfläche darstellt, erfindungsgemäß folgendermaßen berechnet werden:

$$I_{bot} = I_{surf} * e^{-\frac{s_0^{\,2}}{2*\sigma^2}};$$

$$I_{top} = I_{surf} * e^{-\frac{(s_0 - 1)^2}{2*\sigma^2}},$$

sofern gilt:

$$s_0 \geq 2,$$

ansonsten:

$$I_{top} = I_{bot} = I_{surf} * e^{-\frac{1}{2*\sigma^2}},$$

wobei:

$s_0$ -   der Abstand eines Pixel, der maximale Fehlerhelligkeit $I_{bot}$ aufweist, zu dem Pixel, der auf einem Gaußförmigen Grauwertprofil die Helligkeit $I_{surf}$ aufweist, falls die Suchlinie, entlang der das zu untersuchende Grauwertprofil gewonnen wurde, quer zu einer Fehlerkante liegt:

$$s_0 = \sigma * \sqrt{2 * 1n \frac{1}{\eta_0}} \; ;$$

$\eta_0$ -   der Helligkeitsfaktor, der eine allgemeine grundlegende Konstante für die Kantenerkennung darstellt:

$$\eta_0 = \frac{\xi_2}{\xi_1} = e^{-\frac{3}{2}};$$

$\xi_1$ -   der Wendepunktkoeffizient, der die Intensität eines Strahles am Wendepunkt seiner Intensitätsverteilung bestimmt:

$$\xi_1 = e^{-\frac{1}{2}};$$

$\xi_2$ -   der Randpunktkoeffizient, der die Intensität eines Strahles an seinem Rand bestimmt:

$$\xi_2 = e^{-2}.$$

Anschließend müssen alle Pixel im Testbereich untersucht werden. Falls entlang einer Suchlinie, die beispielsweise über alle Zeilen des Testbereiches gelegt werden soll, gilt:

$$I(x) \leq I_{bot} \; ;$$

$$I(x-1) > I_{top},$$

wobei:

$I(x)$ -   die Helligkeit eines beliebigen $x$-Pixels (Pixel an der Stelle $x$);
$I(x-1)$ -   die Helligkeit des vorherigen $x$-$1$-Pixels (Pixel an der Stelle $x$-$1$),

ist $x$-Pixel der Startpunkt des gesuchten Fehlers.
Ab diesem Pixel kann der Fehler weiter mit Hilfe bekannter konventioneller Konturverfolgungsalgorithmen ermittelt werden. Beispielsweise kann diese nach dem Wert $I_{top}$ durchgeführt werden, wobei alle weiteren Pixel, die zum Fehler gehören, eine Helligkeit aufweisen sollen, die unter der minimalen Oberflächenhelligkeit $I_{top}$ liegt (Handbuch der Operatoren für die Bildverarbeitung / Reinhard Klette, Piero Zamperoni. - Braunschweig /Wiesbaden: Friedr. Vieweg & Sohn, 1995).
Das oben beschriebene Verfahren kann mit noch höherer Adaptivität durchgeführt werden. Die nächsthöhere Adaptivitätsstufe kann **gemäß Anspruch 2** erreicht werden, wobei für jeden x-Pixel im Testbereich, für dessen Helligkeit $I(x)$ gilt:

$$I(x) \le I_{bot},$$

wobei:

$$I_{bot} = I_{surf} * e^{-\frac{s_0^2}{2*\sigma^2}};$$

sofern gilt:

$$s_0 \ge 2,$$

ansonsten:

$$I_{bot} = I_{surf} * e^{-\frac{1}{2*\sigma^2}},$$

und damit einen möglichen Startpunkt darstellt, eine lokale Oberflächenhelligkeit $I_{surf\_loc}$ (*local*) aus der unmittelbaren Umgebung vor diesem Pixel ermittelt wird, die beispielsweise den 10-fachen Flächeninhalt des minimalen zu suchenden Fehlers haben soll, und als Ausgangshelligkeit bei der Bestimmung des Startpunktes sowie bei der nachfolgenden Konturverfolgung für die Berechnung der lokalen maximalen Fehlerhelligkeit $I_{bot\_loc}$ und der lokalen minimalen Oberflächenhelligkeit $I_{top\_loc}$ folgendermaßen genutzt wird:

$$I_{bot\_loc} = I_{surf\_loc} * e^{-\frac{s_0^2}{2*\sigma^2}};$$

$$I_{top\_loc} = I_{surf\_loc} * e^{-\frac{(s_0-1)^2}{2*\sigma^2}},$$

sofern gilt:

$$s_0 \ge 2,$$

ansonsten:

$$I_{top\_loc} = I_{bot\_loc} = I_{surf\_loc} * e^{-\frac{1}{2*\sigma^2}}.$$

Falls zusätzlich gilt:

$$I(x) \le I_{bot\_loc};$$

$$I(x - 1) > I_{top\_loc},$$

ist $x$-Pixel der Startpunkt des gesuchten Fehlers und ab diesem Pixel kann der Fehler weiter mit Hilfe bekannter konventioneller Konturverfolgungsmethoden, z.B. nach dem Schwellwert $I_{top\_loc}$, ermittelt werden.

*Gemäß Anspruch 3* kann noch höhere Adaptivitätsstufe erreicht werden, wobei eine lokale Oberflächenhelligkeit $I_{surf\_loc}$ aus der unmittelbaren Umgebung vor jedem zu untersuchenden Pixel im Testbereich ermittelt wird, die beispielsweise den 10-fachen Flächeninhalt des minimalen zu suchenden Fehlers haben soll, und als Ausgangshelligkeit bei der Bestimmung des Startpunktes sowie bei der nachfolgenden Konturverfolgung für die Berechnung der lokalen maximalen Fehlerhelligkeit $I_{bot\_loc}$ sowie der lokalen minimalen Oberflächenhelligkeit $I_{top\_loc}$ folgendermaßen benutzt wird:

$$I_{bot\_loc} = I_{surf\_loc} * e^{-\frac{s_0^2}{2*\sigma^2}};$$

$$I_{top\_loc} = I_{surf\_loc} * e^{-\frac{(s_0-1)^2}{2*\sigma^2}},$$

sofern gilt:

$$s_0 \geq 2,$$

ansonsten:

$$I_{top\_loc} = I_{bot\_loc} = I_{surf\_loc} * e^{-\frac{1}{2*\sigma^2}}.$$

*Gemäß Anspruch 4* kann die minimale Oberflächenhelligkeit $I_{top}$ bzw. die lokale minimale Oberflächenhelligkeit $I_{top\_loc}$ fehlerspezifisch ermittelt wird. Dementsprechend soll für die Erkennung des Startpunktes eines Fehlers, für dessen Helligkeit $I(x)$ gilt:

$$I(x) \leq I_{bot},$$

wobei:

$$I_{bot} = I_{surf} * e^{-\frac{s_0^2}{2*\sigma^2}};$$

bzw.

$$I(x) \leq I_{bot\_loc},$$

wobei:

$$I_{bot\_loc} = I_{surf\_loc} * e^{-\frac{s_0^2}{2*\sigma^2}},$$

sofern gilt:

$$s_0 \geq 2,$$

ansonsten:

$$I_{bot} = I_{surf} * e^{-\frac{1}{2*\sigma^2}},$$

bzw.

$$I_{bot\_loc} = I_{surf\_loc} * e^{-\frac{1}{2*\sigma^2}},$$

die minimale Oberflächenhelligkeit $I_{top}$ bzw. die lokale minimale Oberflächenhelligkeit $I_{top\_loc}$ dynamisch folgendermaßen berechnet werden:

$$I_{top} = I_{surf} * e^{-\frac{s(x)-1)^2}{2*\sigma^2}},$$

falls:

$$s(x) > 2,$$

ansonsten:

$$I_{top} = I_{bot},$$

wobei:

$s(x)$ - der minimale Abstand des $x$-Pixels zu dem Pixel, der auf einem Gaußförmigen Grauwertprofil die Helligkeit $I_{surf}$ aufweisen soll, falls die Suchlinie, entlang der das zu untersuchende Grauwertprofil gewonnen wurde, quer zu einer Fehlerkante liegt:

$$s(x) = \sigma * \sqrt{2*\ln\frac{I_{surf}}{I(x)}} \ ;$$

bzw.

$$I_{top\_loc} = I_{surf\_loc} * e^{-\frac{(s_{loc}(x)-1)^2}{2*\sigma^2}},$$

falls:

$$s_{loc}(x) > 2,$$

ansonsten:

$$I_{top\_loc} = I_{bot\_loc},$$

wobei:

$s_{loc}(x)$ - der minimale Abstand des $x$-Pixels zu dem Pixel, der auf einem Gaußförmigen Grauwertprofil die lokale Helligkeit $I_{surf\_loc}$ aufweisen soll, falls die Suchlinie, entlang der das zu untersuchende Grauwertprofil gewonnen wurde, quer zu einer Fehlerkante liegt:

$$s_{loc}(x) = \sigma^* \sqrt{2^* \ln \frac{I_{surf\_loc}}{I(x)}}.$$

In jedem Fall muss:

$$I(x) > 0,$$

ansonsten wird $I(x)$ als einen zulässigen minimalen Wert, z.B. 1, definiert.

*Gemäß Anspruch 5* kann ein Fehler detektiert werden, indem die maximale Fehlerhelligkeit $I_{bot}$ bzw. die lokale maximale Fehlerhelligkeit $I_{bot\_loc}$ sowie die minimale Oberflächenhelligkeit $I_{top}$ bzw. die lokale minimale Oberflächenhelligkeit $I_{top\_loc}$ anhand der aus dem zu untersuchenden Testbereich gewonnenen Oberflächenhelligkeit $I_{surf}$ bzw. lokalen Oberflächenhelligkeit $I_{surf\_loc}$ sowie der Standardabweichung $\sigma$, die aus dem Grauwertprofil entlang einer Suchlinie ermittelt wurde, welche quer zur einer typischen Kante zwischen der Oberfläche und dem Hintergrund verläuft, folgendermaßen berechnet werden:

$$I_{bot} = I_{surf} * ((1 - \xi_2) * e^{-\frac{s_0^2}{2^* \sigma^2}} + \xi_2);$$

$$I_{top} = I_{surf} * ((1 - \xi_2) * e^{-\frac{(s_0 - 1)^2}{2^* \sigma^2}} + \xi_2)$$

bzw.

$$I_{bot\_loc} = I_{surf\_loc} * ((1 - \xi_{21}) * e^{-\frac{s_0^2}{2^* \sigma^2}} + \xi_2);$$

$$I_{top\_loc} = I_{surf\_loc} * ((1 - \xi_2) * e^{-\frac{(s_0 - 1)^2}{2^* \sigma^2}} + \xi_2),$$

sofern gilt:

$$s_0 \geq 2,$$

ansonsten:

$$I_{top} = I_{bot} = I_{surf} * ((1 - \xi_2) * e^{-\frac{1}{2^* \sigma^2}} + \xi_2);$$

bzw.

$$I_{top\_loc} = I_{bot\_loc} = I_{surf\_loc} * ((1 - \xi_2) * e^{-\frac{1}{2*\sigma^2}} + \xi_2).$$

Ein solcher Fehler kann noch präziser erkannt werden, indem die minimale Oberflächenhelligkeit $I_{top}$ bzw. die lokale minimale Oberflächenhelligkeit $I_{top\_loc}$ fehlerspezifisch ermittelt wird. *Gemäß Anspruch 6* soll sie dynamisch folgendermaßen berechnet werden:

$$I_{top} = I_{surf} * ((1 - \xi_2) * e^{-\frac{(s(x)-1)^2}{2*\sigma^2}} + \xi_2),$$

falls:

$$s(x) > 2,$$

ansonsten:

$$I_{top} = I_{bot},$$

wobei:

$s(x)$ der minimale Abstand des *x*-Pixels zu dem Pixel, der auf einem Gaußförmigen Grauwertprofil die Helligkeit $I_{surf}$ aufweisen soll, falls die Suchlinie, entlang der das zu untersuchende Grauwertprofil gewonnen wurde, quer zu einer Fehlerkante liegt:

$$s(x) = \sigma * \sqrt{2 * \ln \frac{I_{surf}*(1-\xi_2)}{I(x)-I_{surf}*\xi_2}},$$

sofern gilt:

$$I(x) > I_{surf} * \xi_2,$$

ansonsten:

$$s(x) = \sigma * \sqrt{2 * \ln \frac{I_{surf}}{I(x)}} ;$$

bzw.

$$I_{top\_loc} = I_{surf\_loc} * ((1 - \xi_2) * e^{-\frac{(s_{loc}(x)-1)^2}{2*\sigma^2}} + \xi_2),$$

falls:

$$s_{loc}(x) > 2,$$

ansonsten:

$$I_{lop\_loc} = I_{bot\_loc},$$

wobei:

$s_{loc}(x)$ - der minimale Abstand des *x*-Pixels zu dem Pixel, der auf einem Gaußförmigen Grauwertprofil die lokale Helligkeit $I_{surf\_loc}$ aufweisen soll, falls die Suchlinie, entlang der das zu untersuchende Grauwertprofil gewonnen wurde, quer zu einer Fehlerkante liegt:

$$s_{loc}(x) = \sigma * \sqrt{2 * \ln \frac{I_{surf\_loc}*(1-\xi_2)}{I(x)-I_{surf\_loc}*\xi_2}},$$

sofern gilt:

$$I(x) > I_{surf} *\xi_2$$

ansonsten:

$$s_{loc}(x) = \sigma * \sqrt{2 * \ln \frac{I_{surf\_loc}}{I(x)}}.$$

In jedem Fall muss:

$$I(x) > 0,$$

ansonsten wird $I(x)$ als einen zulässigen minimalen Wert, z.B. 1, definiert.

*Gemäß Anspruch 7* wird die Oberflächenhelligkeit $I_{surf}$ bzw. die lokale Oberflächenhelligkeit $I_{surf\_loc}$ als der am häufigsten vorkommende Wert im Bereich von [$I_{sprt}$ - $I_{light}$] des Histogramms des Testbereiches bzw. des Hilfsbereiches ermittelt, wobei der Helligkeitstrennwert $I_{sprt}$ (*separator*) der minimale Wert des zulässigen Oberflächenhelligkeitsbereiches ist:

$$I_{sprt} = I_{max\_abs} * \eta_0;$$

wobei:

$I_{max\_abs}$ - die absolut maximale Helligkeit (z.B. 255 bei Vollaussteuerung und 8-bit Quantisierung), die ein Sensor aufweisen kann;

$I_{light}$ - die maximale Helligkeit aus dem Histogramm des Testbereiches, die als letzte eine Häufigkeit ungleich Null aufweist.

Die Verwendung des Helligkeitstrennwertes $I_{sprt}$ gewährleistet ein sicheres und eindeutiges Verfahren um die Oberflächenhelligkeit $I_{surf}$ bzw. die lokale Oberflächenhelligkeit $I_{surf\_loc}$ auf einem Histogram zu ermitteln, unabhängig davon, wie groß der Maximalwert der Häufigkeiten an den Stelle $I_{surf}$ bzw. $I_{surf\_loc}$ ist, und in welchem Verhältnis dieser Maximalwert zu andern Spitzen in dunklem Bereich von [ 0 - $I_{sprt}$ ] steht, die einen Fehler bzw. Hintergrund aufweisen können.

Das beschriebene adaptive Verfahren erlaubt die Erkennung eines Fehlers, dessen Helligkeit sich von der Oberflächenhelligkeit geringfügig unterscheidet, indem *gemäß Anspruch 8* für die Berechnung der maximalen Fehlerhelligkeit $I_{bot}$ bzw. der lokalen maximalen Fehlerhelligkeit $I_{bot\_loc}$ sowie der minimalen Oberflächenhelligkeit $I_{top}$ bzw. der lokalen minimalen Oberflächenhelligkeit $I_{top\_loc}$ anstatt des Randpunktkoeffizientes $\xi_2$ ein Helligkeitskoeffizient $\xi$ verwendet wird, dessen Größe im Bereich:

$$\xi_2 \leq \xi < 1$$

nach Erfahrungswerten festgestellt werden kann.

*Gemäß Anspruch 9* kann ein solcher Fehler ebenso erkannt werden, indem für die Berechnung sowohl der maximalen Fehlerhelligkeit $I_{bot}$ bzw. lokalen maximalen Fehlerhelligkeit $I_{bot\_loc}$ als auch der minimalen Oberflächenhelligkeit $I_{top}$ bzw. lokalen minimalen Oberflächenhelligkeit $I_{top\_loc}$ sowie der Helligkeitstrennwert $I_{sprt}$ anstatt der Konstante $\eta_0$ für die Kantenerkennung ein Helligkeitsfaktor $\eta$ verwendet wird, dessen Größe im Bereich:

$$\eta_0 \leq \eta < 1$$

nach Erfahrungswerten festgestellt werden kann.

*Gemäß Anspruch 10* kann die Standardabweichung $\sigma$, die unter konkreten Aufnahmebedingungen ganz spezifisch aussehen kann, nach Erfahrungswerten festgestellt und im weiteren als typischer Wert verwendet werden.

*Gemäß Anspruch 11* kann das Grauwertprofil, das entlang einer Suchlinie gewonnen wird, die quer zu einer typischen Kante liegt und evtl. mehrmals angelegt werden kann, sowohl aus einzelner Pixelreihe als auch als Mittelwertkurve aus mehreren entlang zur Suchlinie liegenden benachbarten Pixelreihen, d.h. aus den jeweils senkrecht zur Suchlinie liegenden Pixeln, gebildet werden. Die Anzahl der entlang der Suchlinie liegenden benachbarten Pixelreihen, die je zur Bildung des Grauwertprofils gezogen werden, kann z.B. als drei festgelegt werden. Damit wird eine sichere und genaue Ermittlung der Standardabweichung $\sigma$ gewährleistet.

*Gemäß Anspruch 12* soll nur jede einzelne zu untersuchende Kurve aber nicht das Bild selbst geglättet werden. Damit werden die zu untersuchenden Kurven aus originellen Werten gebildet und nicht schon im Voraus verfälscht.

*Gemäß Anspruch 13* soll morphologische Filterung für die Glättung der zu untersuchenden Kurve verwendet werden. Diese verursacht keine Verfälschung der zu untersuchenden Kurve, die beim Einsatz konventioneller Filterungsmethoden nicht vermeidbar ist, und erlaubt damit eine fehlerfrei Kurvendiskussion.

*Gemäß Anspruch 14* sollen alle Konturpunkte eines Fehlers mit dem gleichen Verfahren wie der Startpunkt ermittelt werden. Damit können alle Konturpunkte mit entsprechender Adaptivität zur lokalen Helligkeitsbedingungen detektiert werden.

Durch das hier genannte Verfahren zur adaptiven Fehlererkennung kann sowohl auf die traditionelle Blobanalyse als auch auf die Texturanalyse zur Erkennung eines Fehlers auf einer strukturierten Oberfläche verzichtet werden.

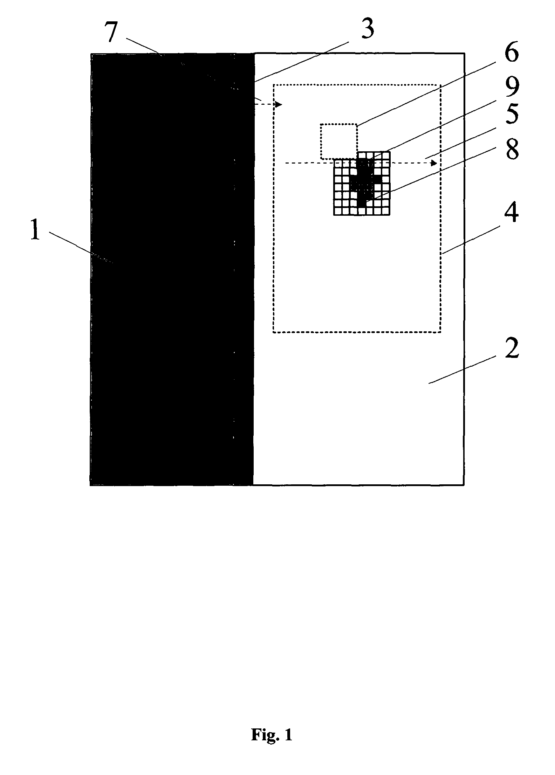

Die Einzelheiten der Erfindung werden in den nachfolgenden Ausführungsbeispielen anhand der **Fig. 1 - 3** erläutert.

Als Beispiel kann angenommen werden, dass einen dunklen Fehler *8* erkannt werden soll, der sich auf einer hellen strukturierten Oberfläche *2,* z.B. Holzoberfläche, befindet, die einen dunklen Hintergrund *1* hat und mit dem sie eine typische Kante *3* bildet **(Fig. 1).**

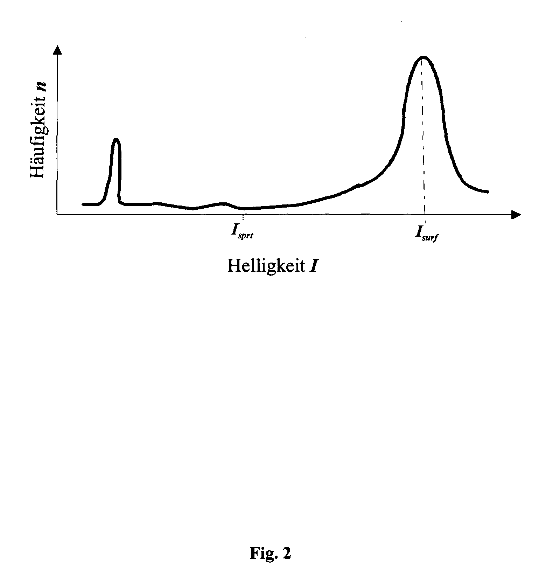

Mit Hilfe eines Histogramms **(Fig. 2),** kann die Oberflächenhelligkeit $I_{surf}$ aus dem Testbereich *4,* ermittelt werden.

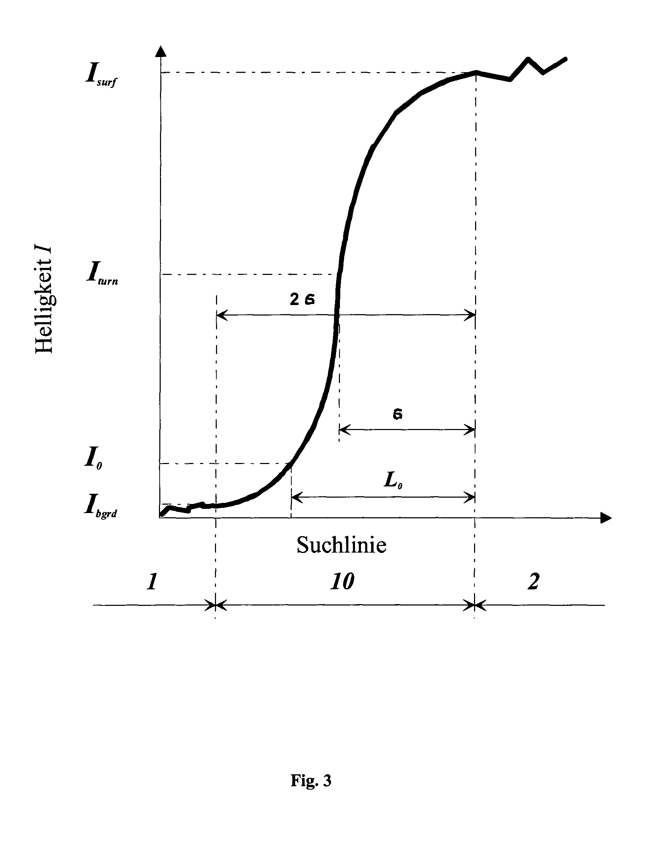

Die Standardabweichung $\sigma$ des Grauwertprofils **(Fig. 3),** das entlang einer Suchlinie *7,* die quer zu einer typischen Kante *3* beispielsweise von dem dunklen Hintergrundbereich *1* zum hellen Oberflächenbereich *2* geht (**Fig. 1**), kann z.B. mit Hilfe der Methode der kleinsten Quadrate ermittelt werden.

Um alle Parameter zu definieren, die für eine adaptive Fehlererkennung benötigt werden, soll zuerst ein Verfahren zur adaptiven Kantensuche beschrieben werden, das der Fehlererkennung zugrunde liegt.

Wie bereits oben begründet, weist das Histogramm sowie das Grauwertprofil ein Gaußsches bzw. ein aus mehreren Gaußverteilungen resultierendes Profil auf. Das bedeutet, dass diese Kurven eine Normalverteilung nach Gauß und damit eine exponentielle Funktion darstellen und deshalb bestimmte Verhältnisse zwischen charakteristischen Punkten besitzen.

Das Grauwertprofil kann als Gaußsches Profil folgendermaßen dargestellt werden:

$$I(x) = I_{surf} * e^{-\frac{x^2}{2*\sigma^2}}, \tag{1}$$

wobei:

$I(x)$ - aktuelle Helligkeit, die der zu untersuchende Punkt auf der Kante aufweist, der sich in der Entfernung $x$ vom Profilmaximum befindet;

$I_{surf}$ - Oberflächenhelligkeit, die auf dem Profilmaximum erstmalig und sicher auftritt;

*x -* die Entfernung des zu untersuchenden Punktes von dem Punkt, der das Profilmaximum aufweist.

Die wichtigsten Punkte auf dem Grauwertprofil für das vorliegenden Verfahren sind Stellen, die eine Entfernung von dem Maximum des Profils haben, die der einfachen und der zweifachen Standardabweichung $\sigma$ des Gaußsches Profils entsprechen **(Fig. 3).**

Die einfache Standardabweichung $\sigma$ ausgehend vom Maximum des Profils zeigt auf einen Punkt, an dem das Grauwertprofil einen Wendepunkt $I_{turn}$ (turn point) besitzt. Hier kann sich damit theoretisch eine Kante **3** befinden:

$$I_{turn} = I_{surf} * \xi_1, \tag{2}$$

wobei:

$I_{surf}$ - die Oberflächenhelligkeit;
$\xi_1$ - der Wendepunktkoeffizient:

$$\xi_1 = e^{-\frac{1}{2}}. \tag{3}$$

Die zweifache Standardabweichung $2^* \sigma$ ausgehend vom Maximum des Profils zeigt auf einen Punkt, an dem das Grauwertprofil die Intensität von dem Strahlrand hat und sich damit der Hintergrund **1** befindet:

$$I_{bgrd} = I_{surf} * \xi_2, \tag{4}$$

wobei: $\xi_2$ - der Randpunktkoeffizient:

$$\xi_2 = e^{-2}. \tag{5}$$

Der Abstand zwischen den Punkten, welche die Werte $I_{turn}$ und $I_{bgrd}$ aufweisen, entspricht ebenso der Standardabweichung $\sigma$ und ist vom jeweiligen Grauwertprofil (**Fig. 3**) streng abhängig. Das Verhältnis zwischen diesen Werten ist jedoch für alle möglichen Grauwertprofile, die über eine Kante **3** verlaufen, konstant und stellt einen minimalen Helligkeitsfaktor $\eta_0$ dar. Nach (2) bis (5) gilt:

$$\eta_0 = \frac{I_{bgrd}}{I_{turn}} = \frac{\xi_2}{\xi_1} = e^{-\frac{3}{2}}. \tag{6}$$

Damit stellt der Helligkeitsfaktor $\eta_0$ ein minimales Verhältnis zwischen den Helligkeiten der erst möglichen Kantenposition **3** und dem Hintergrund **1** dar:

Aus dem Grauwertprofil **(Fig. 3)** kann mit Hilfe bekannter konventioneller Rechenmethoden, z.B. die Methode der kleinsten Quadrate, die Standardabweichung $\sigma$ dieses Profils als Gaußschen Profils ermittelt werden.

Um die Position der zu untersuchenden Kante **3** auf dem Grauwertprofil finden zu können, soll das Grauwertprofil mit einer Teststrecke abgesucht werden.

Die Länge dieser Teststrecke $L_0$ darf dabei nicht kleiner als der Abstand zwischen dem Wendepunkt ($I_{turn}$) und dem Randpunkt (Hintergrundhelligkeit $I_{bgrd}$) sein, was der Standardabweichung $\sigma$ des Grauwertprofils entspricht. Gleichzeitig darf die Teststrecke $L_0$ die doppelte Größe der Standardabweichung $\sigma$ nicht überschreiten. Sonst wird die Teststrecke größer als der gesamte Übergangsbereich **10** zwischen dem Hintergrund **1** und der Oberfläche **2** (**Fig. 3**). Dann kann die Kantensuche nur nach der kantenspezifischen minimalen Helligkeitssteigung $\Delta I_0$, ohne Berücksichtigung der kantenspezifischen minimalen Helligkeit $I_0$, die eine wichtige Bedingung für die adaptive Kantenerkennung darstellt, durchgeführt werden. Deshalb soll gelten:

$$\sigma < L_0 < 2^* \sigma. \tag{7}$$

Dies bedeutet, dass eine Kante **3** anwesend ist, solange die folgenden Bedingungen innerhalb der Teststrecke $L_0$ erfüllt sind:

$$I_{max} \geq I_0 ; \qquad (8)$$

$$\Delta I = I_{max} - I_{min} \geq \Delta I_0, \qquad (9)$$

wobei:

$$\frac{I_{min}}{I_{max}} = \eta \geq \eta_0, \qquad (10)$$

$I_{max}$ - die lokale maximale Helligkeit innerhalb der Teststrecke $L_0$ ;
$I_{min}$ - die lokale minimale Helligkeit innerhalb der Teststrecke $L_0$ ;
$\Delta I$- die lokale Helligkeitssteigung innerhalb der Teststrecke $L_0$ ;
$\eta$ - der lokale Helligkeitsfaktor innerhalb der Teststrecke $L_0$ .

Zur Bestimmung der Parameter $I_0$ und $\Delta I_0$ werden folgende Grenzfälle betrachtet. Wenn der Endpunkt der Teststrecke $L_0$ bereits die Oberfläche **2** erreicht hat:

$$I_{max} = I_{surf}, \qquad (11)$$

ist die Kante **3** noch immer innerhalb dieser Teststrecke. Dann folgt aus (10):

$$I_{min} = I_{max} * \eta_0. \qquad (12)$$

Die lokale minimale Helligkeit $I_{min}$ entspricht dabei noch nicht der Oberflächenhelligkeit ($I_{min} < I_{surf}$) aber nicht mehr der Hintergrundhelligkeit ($I_{min} > I_{bgrd}$). Sie weist nun auf einen Übergangsbereich **10** zwischen dem Hintergrund **1** und der Oberfläche **2,** in dem sich eine Kante **3** befinden kann, und bestimmt damit die minimale Helligkeit $I_0$. Die kantenspezifische minimale Helligkeit $I_0$ kann dann mit Hilfe von (11) und (12) folgendermaßen berechnet werden:

$$I_0 = I_{surf} * \eta_0, \qquad (13)$$

Befindet sich der Endpunkt der Teststrecke $L_0$ bereits in einer Position, die der Helligkeit $I_0$ entspricht, und der Startpunkt noch auf dem Hintergrund **1,** soll im Falle einer wirklichen Kante erst die Bedingung (9) erfüllt sein. D. h. die Suche nach einer Kante darf nur dann angefangen werden, wenn die lokale Helligkeitssteigung $\Delta I$ innerhalb der Teststrecke $L_0$ die kantenspezifische minimale Helligkeitssteigung $\Delta I_0$ erreicht. Unter der Annahme, dass der Startpunkt der Teststrecke $L_0$ sich im günstigsten Fall in einer Position befindet, die den Helligkeitswert null aufweist, kann die kantenspezifische minimale Helligkeitssteigung $\Delta I_0$, die die Startbedingungen für die Kantensuche bestimmt, folgendermaßen definiert werden:

$$\Delta I_0 = I_0 - 0 = I_0 = I_{surf} * \eta_0. \qquad (14)$$

Damit kann eine Kante **3** zwischen den zwei folgenden Positionen und nur dort vorhanden sein. Die erste Position entspricht dem Endpunkt der Teststrecke $L_0$ , wenn die Bedingungen (8) und (9) zum ersten Mal innerhalb der Teststrecke $L_0$ gleichzeitig erfüllt sind. Die zweite Position entspricht dem Startpunkt der Teststrecke $L_0$ , wenn die Bedingungen (8) und (9) zum letzten Mal innerhalb der Teststrecke $L_0$ gleichzeitig erfüllt sind.

Nun können die Schwellwerte $I_{bot}$ und $I_{top}$ für die Detektierung des Startpunktes **9** eines Fehlers **8 (Fig. 1)** definiert werden.

Falls gilt:

$$I_{bgrd} \leq I_{surf} * \xi_2,$$

unterscheidet sich die Oberflächenhelligkeit $I_{surf}$ von der Hintergrundhelligkeit $I_{bgrd}$ genügend, um eine zwischen der Oberfläche *2* und dem Hintergrund *1* entstehende Kante *3* sicher erkennen zu können.

Der zu suchende Fehler *8* stellt eigentlich eine Beschädigung der Oberfläche *2* dar. Diese bildet die auf einem Licht-und-Schatten-Bild genauso wie der Hintergrund *1* einen dunklen Bereich auf der hellen Oberfläche *2.* Ebenso hat der Fehler *8* mit der Oberfläche *2* auch eine Kante. Aus diesem Grund kann die kantenspezifische minimale Helligkeit $I_0$ nach (13) als Schwellwert $I_{bot}$, der für die kantenbasierte Segmentierung des Fehlers *8* als maximale Fehlerhelligkeit dient, angenommen werden:

$$I_{bot} = I_{surf} * \eta_0. \tag{15}$$

Falls entlang einer Suchlinie *5* im Testbereich *4* für einen beliebigen *x*-Pixel, der eine Helligkeit $I(x)$ aufweist, gilt:

$$I(x) \leq I_{bot}, \tag{16}$$

ist auf diesem Pixel möglicherweise einen Fehler abgebildet. Soll dieser Pixel tatsächlich ein Startpunkt *9* des Fehlers *8* sein, geht die Suchlinie *5* durch eine Kante zwischen der Oberfläche *2* und dem Fehler *8* **(Fig. 1).** In dem Fall muss das Grauwertprofil, das entlang der Suchlinie *5* gewonnen wurde, die gleiche bzw. größere Steigung aufweisen, als Grauwertprofil, das an einer typischen Kante *3* entlang der Suchlinie *7* gewonnen wurde. Dann gilt nach (1) für dieses Gaußförmigen Grauwertprofil:

$$I_{bot} = I_{surf} * e^{-\frac{s_0^2}{2*\sigma^2}}, \tag{17}$$

wobei:

$s_0$ -   der Abstand eines Pixel, der maximale Fehlerhelligkeit $I_{bot}$ aufweist, zu dem Pixel, der auf einem Gaußförmigen Grauwertprofil die Helligkeit $I_{surf}$ aufweist, falls die Suchlinie *5*, entlang der das zu untersuchende Grauwertprofil gewonnen wurde, quer zu einer Fehlerkante verläuft.

Aus (15) und (17) folgt:

$$s_0 = \sigma * \sqrt{2 * \ln \frac{1}{\eta_0}}. \tag{18}$$

Die Helligkeit $I(x-1)$ des vorherigen *x-1*-Pixels soll dementsprechend folgendermaßen aussehen:

$$I(x-1) = I_{surf} * e^{-\frac{(s_0-1)^2}{2*\sigma^2}}. \tag{19}$$

Nun, um es prüfen zu können, ob die Suchlinie *5* quer zu einer Kante zwischen der Oberfläche *2* und dem Fehler *8* geht, kann die minimale Oberflächenhelligkeit $I_{top}$ aus (19) folgendermaßen definiert werden:

$$I_{top} = I_{surf} * e^{-\frac{(s_0-1)^2}{2*\sigma^2}}. \tag{20}$$

sofern gilt:

$$s_0 \geq 2,$$

ansonsten:

$$s_0 = 1,$$

weil:

$$I_{bot} \leq I_{top} < I_{surf}.$$

In dem Fall werden die beide Schwellwerte $I_{bot}$ und $I_{top}$ folgendermaßen definiert:

$$I_{top} = I_{bot} = I_{surf} * e^{-\frac{1}{2*\sigma^2}}, \qquad (21)$$

Nun, falls für *x-1*-Pixel gilt:

$$I(x - 1) > I_{top}, \qquad (22)$$

ist der x-Pixel tatsächlich der Startpunkt 9 des gesuchten Fehlers **8** (**Fig. 1**). Ab diesem Pixel soll der Fehler weiter mit Hilfe bekannter konventioneller Konturverfolgungsmethoden ermittelt werden.
Ein Fehler **8** kann noch präziser detektiert werden, indem die minimale Oberflächenhelligkeit $I_{top}$ fehlerspezifisch ermittelt wird. Falls für einen *x*-Punkt mit der Helligkeit $I(x)$ gilt:

$$I(x) \leq I_{bot},$$

kann die minimale Oberflächenhelligkeit $I_{top}$ von der Helligkeit $I(x)$ her patentgemäß dynamisch folgendermaßen berechnet werden:

$$I_{top} = I_{surf} * e^{-\frac{s(x)-1)^2}{2*\sigma^2}}, \qquad (23)$$

falls:

$$s(x) > 2,$$

ansonsten:

$$I_{top} = I_{bot}, \qquad (24)$$

wobei:

$s(x)$ -  der minimale Abstand des *x*-Pixels zu dem Pixel, der auf einem Gaußförmigen Grauwertprofil die Helligkeit $I_{surf}$ aufweisen soll, falls die Suchlinie **5,** entlang der das zu untersuchende Grauwertprofil gewonnen wurde, quer zu einer Fehlerkante liegt:

$$s(x) = \sigma^* \sqrt{2^*1\mathrm{n}\frac{I_{surf}}{I(x)}}. \tag{25}$$

Dabei muss:

$$I(x) > 0,$$

ansonsten wird $I(x)$ mit einem zulässigen minimalen Wert, z.B. 1, ersetzt.

Die Schwellwerte $I_{bot}$ und $I_{top}$ für die Detektierung des Startpunktes **9** von einem Fehler **8** können so definiert werden, dass die Helligkeit des zu suchenden Fehlers dabei berücksichtigt wird, als ob sie die Hintergrundhelligkeit $I_{bgrd}$ für die Fehlerkantenerkennung wäre. In dem Fall kann der Schwellwert $I_{bot}$ folgendermaßen definiert werden:

$$I_{bot} = (I_{surf} - I_{bgrd})^* \eta_0 + I_{bgrd}. \tag{26}$$

Nach (4) und (26):

$$I_{bot} = I_{surf}^* ((1 - \xi_2)^* \eta_0 + \xi_2). \tag{27}$$

Mit der Berücksichtigung von (18) für die Schwellwerte $I_{bot}$ und $I_{top}$ gilt:

$$I_{bot} = I_{surf}^* ((1 - \xi_2)^* e^{-\frac{s_0^2}{2^*\sigma^2}} + \xi_2); \tag{28}$$

$$I_{top} = I_{surf}^* ((1 - \xi_2)^* e^{-\frac{(s_0-1)^2}{2^*\sigma^2}} + \xi_2) \tag{29}$$

falls:

$$s_0 \geq 2,$$

ansonsten:

$$I_{top} = I_{bot} = I_{surf}^* ((1 - \xi_2)^* e^{-\frac{1}{2^*\sigma^2}} + \xi_2). \tag{30}$$

Um ein Fehler **8** noch präziser erkennen zu können, soll die minimale Oberflächenhelligkeit $I_{top}$ fehlerspezifisch ermittelt werden.

Nach (23) und (29):

$$I_{top} = I_{surf}^* ((1 - \xi_2)^* e^{-\frac{(s_0-1)^2}{2^*\sigma^2}} + \xi_2), \tag{31}$$

falls:

$$s_0 \geq 2,$$

ansonsten:

$$I_{top} = I_{bot},$$

wobei nach (25) und (29):

$$d_0 = \sigma * \sqrt{2 * \ln \frac{I_{surf}*(1 - \xi_2)}{I(x) - I_{surf}*\xi_2}} \; ; \qquad (32)$$

sofern gilt:

$$I(x) > I_{surf} * \xi_2,$$

ansonsten nach (25):

$$s(x) = \sigma * \sqrt{2 * \ln \frac{I_{surf}}{I(x)}} \; ;$$

Dabei muss:

$$I(x) > 0,$$

ansonsten wird $I(x)$ mit einem zulässigen minimalen Wert, z.B. 1, ersetzt.

Die lokalen Schwellwerte $I_{bot\_loc}$ und $I_{top\_loc}$ lassen sich analog zu den entsprechenden Schwellwerten $I_{bot}$ und $I_{top}$ berechnen, damit anstatt der Oberflächenhelligkeit $I_{surf}$ eine lokale Oberflächenhelligkeit $I_{surf\_loc}$ verwendet werden kann.

Es kann dabei wichtig sein, dass die Ermittlung der Oberflächenhelligkeit $I_{surf}$ bzw. der lokalen Oberflächenhelligkeit $I_{surf\_loc}$ aus dem Histogramm des Testbereiches **4** bzw. des Hilfsbereiches **6** mit Hilfe eines Helligkeitstrennwerts $I_{sprt}$ durchgeführt werden kann. Diese soll für die Abtrennung des entsprechenden Bereiches von $[I_{sprt} - I_{light}]$ auf dem Histogramm genutzt werden, damit die Oberflächenhelligkeit $I_{surf}$ eindeutig ermittelt werden kann (**Fig. 2**).

Die obere Grenze der Hintergrundhelligkeit $I_{bgrd\_max}$ kann nach (12) folgendermaßen definiert werden:

$$I_{bgrd\_max} = I_{surf} * \eta_0. \qquad (33)$$

Die Hintergrundhelligkeit $I_{bgrd\_max}$ nach (33) weist einen Wert auf, der gleichzeitig die Objekthelligkeit $I_{surf}$ von unten begrenzt. Damit kann die absolut minimale zulässige Objekthelligkeit $I_{surf\_min}$, die nie erreicht werden darf, folgendermaßen definiert werden:

$$I_{surf\_min} = I_{max\_abs} * \eta_0, \qquad (34)$$

wobei:

$I_{max\_abs}$ - die absolut maximale Helligkeit der Oberfläche **2**, die ein Aufnahmesensor maximal aufweisen kann (z. B. 255 bei Vollaussteuerung und 8-bit Quantisiereung des Sensors).

Dieser Wert kann auf einem Histogramm **(Fig. 2)** als Helligkeitstrennwert $I_{sprt}$ für die Trennung zwischen dem Oberflächenbereich und dem restlichem Bereich, der die Fehler bzw. der Hintergrund aufweist, verwendet werden:

$$I_{sprt} = I_{\max\_abs} * \eta_0, \tag{35}$$

Damit lässt das oben beschriebene adaptive Verfahren einen auf einer strukturierten Oberfläche vorhandenen Fehler explizit erkennen.

Bezugszeichenliste

**1** Hintergrund
**2** Oberfläche
**3** Kante zwischen dem Hintergrund und der Oberfläche
**4** Testbereich auf der Oberfläche
**5** Suchlinie im Testbereich auf der Oberfläche
**6** Unmittelbare Umgebung vor dem zu untersuchenden Pixel
**7** Suchlinie quer zur Kante zwischen dem Hintergrund und der Oberfläche
**8** Fehler auf der Oberfläche
**9** Startpunkt des Fehlers auf der Oberfläche
**10** Übergangsbereich zwischen dem Hintergrund und der Oberfläche

**Patentansprüche**

**1.** Verfahren zur adaptiven Erkennung eines Fehlers **8** auf einer strukturierten Oberfläche **2,** z.B. einer Holzoberfläche, der auf einem mit einem Kamerasensor aufgenommenen Bild mit Hilfe konturverfolgungsbasierter Segmentierung (Blobanalyse) gesucht wird, wobei eine maximale Fehlerhelligkeit $I_{bot}$ (*bottom*) und eine minimale Oberflächenhelligkeit $I_{top}$ (*top*) für die Detektierung des Startpunktes **9** des gesuchten Fehlers **8** (Blob) genutzt werden, **dadurch gekennzeichnet, dass** die Schwellwerte $I_{bot}$ und $I_{top}$ angepasst zu lokalen Helligkeitsbedingungen dynamisch aus dem Histogramm des Testbereichs **4** zusammen mit dem Grauwertprofil entlang einer Suchlinie **5** im Testbereich **4** ermittelt werden, wobei das Histogramm und das Grauwertprofil als Gaußverteilungen behandelt werden.
Patentgemäß ist die Erkennung eines Fehlers folgendermaßen durchzuführen.
Aus dem Histogramm des Testbereiches **4** soll die Oberflächenhelligkeit $I_{surf}$ (*surface*) als der am häufigsten vorkommende Wert des Histogramms ermittelt werden.
Aus dem Grauwertprofil entlang einer Suchlinie **7,** die quer zur einer typischen Kante **3** zwischen der Oberfläche **2** und dem Hintergrund **1** verläuft, soll die Standardabweichung σ mit Hilfe bekannter konventionellen Rechenmethoden, z.B. die Methode der kleinsten Quadrate, ermittelt werden. Diese wird als kantencharakteristischer Parameter für die Berechnung der maximalen Fehlerhelligkeit $I_{bot}$ und der minimalen Oberflächenhelligkeit $I_{top}$ dienen.
Falls die Suchlinie **5**, entlang der das zu untersuchende Grauwertprofil gewonnen wurde, quer zu einer Kante des Fehlers liegt, kann der Abstand $s_0$ eines Pixels, der die maximale Fehlerhelligkeit $I_{bot}$ aufweist, zu dem Pixel, der auf einem Gaußförmigen Grauwertprofil die Helligkeit $I_{surf}$ aufweist, folgendermaßen definiert werden:

$$s_0 = \sigma * \sqrt{2*\ln\frac{1}{\eta_0}} \ ,$$

wobei:

$\eta_0$ - der Helligkeitsfaktor, der eine allgemeine grundlegende Konstante für die Kantenerkennung darstellt:

$$\eta_0 = \frac{\xi_2}{\xi_1} = e^{-\frac{3}{2}} \ ;$$

$\xi_1$ - der Wendepunktkoeffizient, der die Intensität eines Strahles am Wendepunkt seiner Intensitäts-

verteilung bestimmt:

$$\xi_1 = e^{-\frac{1}{2}};$$

$\xi_2$ -  der Randpunktkoeffizient, der die Intensität eines Strahles an seinem Rand bestimmt:

$$\xi_2 = e^{-2}.$$

Anhand der gewonnenen Oberflächenhelligkeit $I_{surf}$ und des Abstandes $s_0$ kann die maximale Fehlerhelligkeit $I_{bot}$, die die größte zulässige Helligkeit eines Fehlers darstellt, sowie die minimale Oberflächenhelligkeit $I_{top}$, die die kleinste zulässige Helligkeit der Oberfläche darstellt, erfindungsgemäß folgendermaßen berechnet werden:

$$I_{bot} = I_{surf} * e^{-\frac{s_0^2}{2*\sigma^2}};$$

$$I_{top} = I_{surf} * e^{-\frac{(s_0-1)^2}{2*\sigma^2}},$$

sofern gilt:

$$s_0 \geq 2,$$

ansonsten:

$$I_{top} = I_{bot} = I_{surf} * e^{-\frac{1}{2*\sigma^2}}.$$

Anschließend müssen alle Pixel im Testbereich **4** untersucht werden. Falls entlang einer Suchlinie **5,** die beispielsweise über alle Zeilen des Testbereiches gelegt werden soll, gilt:

$$I(x) \leq I_{bot} ;$$

$$I(x - 1) > I_{top},$$

wobei:

$I(x)$ -  die Helligkeit eines beliebigen $x$-Pixels (Pixel an der Stelle $x$);
$I(x$ -1) die Helligkeit des vorherigen $x$-$1$-Pixels (Pixel an der Stelle $x$-$1$),

ist $x$-Pixel der Startpunkt **9** des gesuchten Fehlers **8.**
Ab diesem Pixel kann der Fehler **8** weiter mit Hilfe bekannter konventioneller Konturverfolgungsmethoden, z.B. nach dem Schwellwert $I_{top}$, ermittelt werden, wobei alle Pixel, die zum Fehler gehören, eine Helligkeit aufweisen sollen, die unter der minimalen Oberflächenhelligkeit $I_{top}$ liegt.

**2.** Verfahren nach Anspruch 1, **dadurch gekennzeichnet, dass** für jeden $x$-Pixel im Testbereich **4,** für dessen Helligkeit $I(x)$ gilt:

$$I(x) \leq I_{bot},$$

wobei:

$$I_{bot} = I_{surf} * e^{-\frac{s_0{}^2}{2*\sigma^2}},$$

sofern gilt:

$$s_0 \geq 2,$$

ansonsten:

$$I_{bot} = I_{surf} * e^{-\frac{1}{2*\sigma^2}},$$

und damit einen möglichen Startpunkt darstellt, eine lokale Oberflächenhelligkeit $I_{surf\_loc}$ (*local*) aus der unmittelbaren Umgebung **6** vor diesem Pixel ermittelt wird, die beispielsweise den 10-fachen Flächeninhalt des minimalen zu suchenden Fehlers haben soll, und als Ausgangshelligkeit bei der Bestimmung des Startpunktes **9** sowie bei der nachfolgenden Konturverfolgung für die Berechnung der lokalen maximalen Fehlerhelligkeit $I_{bot\_loc}$ und der lokalen minimalen Oberflächenhelligkeit $I_{top\_loc}$ folgendermaßen genutzt wird:

$$I_{bot\_loc} = I_{surf\_loc} * e^{-\frac{s_0{}^2}{2*\sigma^2}};$$

$$I_{top\_loc} = I_{surf\_loc} * e^{-\frac{(s_0-1)^2}{2*\sigma^2}},$$

sofern gilt:

$$s_0 \geq 2,$$

ansonsten:

$$I_{top\_loc} = I_{bot\_loc} = I_{surf\_loc} * e^{-\frac{1}{2*\sigma^2}}.$$

Falls zusätzlich gilt:

$$I(x) \leq I_{bot\_loc};$$

$$I(x - 1) > I_{top\_loc},$$

ist x-Pixel der Startpunkt **9** des gesuchten Fehlers **8** und ab diesem Pixel kann der Fehler **8** weiter mit Hilfe bekannter konventioneller Konturverfolgungsmethoden, z.B. nach dem Schwellwert $I_{top\_loc}$, ermittelt werden.

3.  Verfahren nach Anspruch 1 und 2, **dadurch gekennzeichnet, dass** eine lokale Oberflächenhelligkeit $I_{surf\_loc}$ aus der unmittelbaren Umgebung **6** vor jedem zu untersuchenden Pixel im Testbereich **4** ermittelt wird, die beispielsweise den 10-fachen Flächeninhalt des minimalen zu suchenden Fehlers haben soll, und als Ausgangshelligkeit bei der Bestimmung des Startpunktes **9** sowie bei der nachfolgenden Konturverfolgung für die Berechnung der lokalen maximalen Fehlerhelligkeit $I_{bot\_loc}$ sowie der lokalen minimalen Oberflächenhelligkeit $I_{top\_loc}$ folgendermaßen benutzt wird:

$$I_{bot\_loc} = I_{surf\_loc} * e^{-\frac{s_0^2}{2*\sigma^2}};$$

$$I_{top\_loc} = I_{surf\_loc} * e^{-\frac{(s_0-1)^2}{2*\sigma^2}},$$

sofern gilt:

$$s_0 \geq 2,$$

ansonsten:

$$I_{top\_loc} = I_{bot\_loc} = I_{surf\_loc} * e^{-\frac{1}{2*\sigma^2}}.$$

4.  Verfahren nach Anspruch 1 bis 3, **dadurch gekennzeichnet, dass** der Startpunkt **9** eines Fehlers **8** auf einer Oberfläche **2,** für dessen Helligkeit $I(x)$ gilt:

$$I(x) \leq I_{bot},$$

wobei:

$$I_{bot} = I_{surf} * e^{-\frac{s_0^2}{2*\sigma^2}};$$

bzw.

$$I(x) \leq I_{bot\_loc},$$

wobei:

$$I_{bot\_loc} = I_{surf\_loc} * e^{-\frac{s_0^2}{2*\sigma^2}},$$

sofern gilt:

$$s_0 \geq 2,$$

ansonsten:

$$I_{bot} = I_{surf} * e^{-\frac{1}{2*\sigma^2}},$$

bzw.

$$I_{bot\_loc} = I_{surf\_loc} * e^{-\frac{1}{2*\sigma^2}},$$

detektiert werden kann, indem die minimale Oberflächenhelligkeit $I_{top}$ bzw. die lokale minimale Oberflächenhelligkeit $I_{top\_loc}$ fehlerspezifisch ermittelt wird.

Patentgemäß soll sie dynamisch folgendermaßen berechnet werden:

$$I_{top} = I_{surf} * e^{-\frac{s(x)-1)^2}{2*\sigma^2}},$$

falls:

$$s(x) > 2,$$

ansonsten:

$$I_{top} = I_{bot},$$

wobei:

$s(x)$ - der minimale Abstand des x-Pixels zu dem Pixel, der auf einem Gaußförmigen Grauwertprofil die Helligkeit $I_{surf}$ aufweisen soll, falls die Suchlinie **5,** entlang der das zu untersuchende Grauwertprofil gewonnen wurde, quer zu einer Fehlerkante liegt:

$$s(x) = \sigma * \sqrt{2*\ln\frac{I_{surf}}{I(x)}} ;$$

bzw.

$$I_{top\_loc} = I_{surf\_loc} * e^{-\frac{(s_{loc}(x)-1)^2}{2*\sigma^2}},$$

falls:

$$s_{loc}(x) > 2,$$

ansonsten:

$$I_{top\_loc} = I_{bot\_loc},$$

wobei:

$s_{loc}$ (*x*) - der minimale Abstand des *x*-Pixels zu dem Pixel, der auf einem Gaußförmigen Grauwertprofil die lokale Helligkeit $I_{surf\_loc}$ aufweisen soll, falls die Suchlinie **5**, entlang der das zu untersuchende Grauwertprofil gewonnen wurde, quer zu einer Fehlerkante liegt:

$$s_{loc}(x) = \sigma * \sqrt{2 * 1n \frac{I_{surf\_loc}}{I(x)}};$$

sofern gilt:

$$I(x) > 0 \,,$$

ansonsten muss $I(x)$ mit einem zulässigen minimalen Wert, z.B. 1, ersetzt werden.

**5.** Verfahren nach Anspruch 1 bis 3, **dadurch gekennzeichnet, dass** der Startpunkt **9** eines Fehlers **8** auf einer Oberfläche 2 detektiert werden kann, indem die maximale Fehlerhelligkeit $I_{bot}$ bzw. die lokale maximale Fehlerhelligkeit $I_{bot\_loc}$ sowie die minimale Oberflächenhelligkeit $I_{top}$ bzw. die lokale minimale Oberflächenhelligkeit $I_{top\_loc}$ anhand der aus dem zu untersuchenden Testbereich **4** gewonnenen Oberflächenhelligkeit $I_{surf}$ bzw. lokalen Oberflächenhelligkeit $I_{surf\_loc}$ sowie der Standardabweichung σ, die aus dem Grauwertprofil entlang einer Suchlinie **7** ermittelt wurde, welche quer zur einer typischen Kante **3** zwischen der Oberfläche **2** und dem Hintergrund **1** verläuft, folgendermaßen berechnet werden:

$$I_{bot} = I_{surf} * ((1 - \xi_2) * e^{-\frac{s_0^2}{2*\sigma^2}} + \xi_2);$$

$$I_{top} = I_{surf} * ((1 - \xi_2) * e^{-\frac{(s_0 - 1)^2}{2*\sigma^2}} + \xi_2)$$

bzw.

$$I_{bot\_loc} = I_{surf\_loc} * ((1 - \xi_2) * e^{-\frac{s_0^2}{2*\sigma^2}} + \xi_2);$$

$$I_{top\_loc} = I_{surf\_loc} * ((1 - \xi_2) * e^{-\frac{(s_0 - 1)^2}{2*\sigma^2}} + \xi_2),$$

sofern gilt:

$$s_0 \geq 2,$$

ansonsten:

$$I_{top} = I_{bot} = I_{surf} * ((1 - \xi_2) * e^{-\frac{1}{2*\sigma^2}} + \xi_2);$$

bzw.

$$I_{top\_loc} = I_{bot\_loc} = I_{surf\_loc} * ((1 - \xi_2) * e^{-\frac{1}{2*\sigma^2}} + \xi_2).$$

6. Verfahren nach Anspruch 1 bis 5, **dadurch gekennzeichnet, dass** der Startpunkt *9* eines Fehlers *8* auf einer Oberfläche *2* detektiert werden kann, indem die minimale Oberflächenhelligkeit $I_{top}$ bzw. die lokale minimale Oberflächenhelligkeit $I_{top\_loc}$ fehlerspezifisch ermittelt wird.
Patentgemäß soll sie dynamisch folgendermaßen berechnet werden:

$$I_{top} = I_{surf} * ((1 - \xi_2) * e^{-\frac{(s(x)-1)^2}{2*\sigma^2}} + \xi_2),$$

falls:

$$s(x) > 2,$$

ansonsten:

$$I_{top} = I_{bot},$$

wobei:

$s(x)$ - der minimale Abstand des *x*-Pixels zu dem Pixel, der auf einem Gaußförmigen Grauwertprofil die Helligkeit $I_{surf}$ aufweisen soll, falls die Suchlinie *5,* entlang der das zu untersuchende Grauwertprofil gewonnen wurde, quer zu einer Fehlerkante liegt:

$$s(x) = \sigma * \sqrt{2 * 1n \frac{I_{surf}*(1-\xi_2)}{I(x)-I_{surf}*\xi_2}},$$

sofern gilt:

$$I(x) > I_{surf} * \xi_2,$$

ansonsten:

$$s(x) = \sigma * \sqrt{2 * 1n \frac{I_{surf}}{I(x)}} ;$$

bzw.

$$I_{top\_loc} = I_{surf\_loc} * ((1 - \xi_2) * e^{-\frac{(s_{loc}(x)-1)^2}{2*\sigma^2}} + \xi_2),$$

falls:

$$s_{loc}(x) > 2,$$

ansonsten:

$$I_{lop\_loc} = I_{bot\_loc},$$

wobei:

$s_{loc}(x)$ - der minimale Abstand des $x$-Pixels zu dem Pixel, der auf einem Gaußförmigen Grauwertprofil die lokale Helligkeit $I_{surf\_loc}$ aufweisen soll, falls die Suchlinie **5,** der entlang das zu untersuchende Grauwertprofil gewonnen wurde, quer zu einer Fehlerkante liegt:

$$s_{loc}(x) = \sigma * \sqrt{2 * 1n \, \frac{I_{surf\_loc} * (1-\xi_2)}{I(x) - I_{surf\_loc} * \xi_2}},$$

sofern gilt:

$$I(x) > I_{surf} * \xi_2$$

ansonsten:

$$s_{loc}(x) = \sigma * \sqrt{2 * 1n \frac{I_{surf\_loc}}{I(x)}};$$

sofern gilt:

$$I(x) > 0,$$

ansonsten muss $I(x)$ mit einem zulässigen minimalen Wert, z.B. 1, ersetzt werden.

**7.** Verfahren nach Anspruch 1 bis 6, **dadurch gekennzeichnet, dass** die Oberflächenhelligkeit $I_{surf}$ bzw. die lokale Oberflächenhelligkeit $I_{surf\_loc}$ als der am häufigsten vorkommende Wert im Bereich von [ $I_{sprt}$ - $I_{light}$ ] des Histogramms des Testbereiches **4** bzw. des Hilfsbereiches **6** ermittelt wird, wobei der Helligkeitstrennwert $I_{sprt}$ *(separator)* der minimale Wert des zulässigen Oberflächenhelligkeitsbereiches ist:

$$I_{sprt} = I_{max\_abs} * \eta_0;$$

wobei:

$I_{max\_abs}$ - die absolut maximale Helligkeit (z.B. 255 bei Vollaussteuerung und 8-bit Quantisierung), die ein Sensor aufweisen kann,

$I_{light}$ - die maximale Helligkeit aus dem Histogramm des Testbereiches, die als letzte eine Häufigkeit ungleich Null aufweist.

**8.** Verfahren nach Anspruch 5 und 6, **dadurch gekennzeichnet, dass** der Startpunkt **9** eines Fehlers **8** auf einer Oberfläche **2**, dessen Helligkeit sich von der Oberflächenhelligkeit geringfügig unterscheidet, erkannt werden kann, indem für die Berechnung der maximalen Fehlerhelligkeit $I_{bot}$ bzw. der lokalen maximalen Fehlerhelligkeit $I_{bot\_loc}$ sowie der minimalen Oberflächenhelligkeit $I_{top}$ bzw. der lokalen minimalen Oberflächenhelligkeit $I_{top\_loc}$ anstatt des Randpunktkoeffizientes $\xi_2$ ein Helligkeitskoeffizient $\xi$ verwendet wird, dessen Größe im Bereich:

$$\xi_2 \leq \xi < 1$$

nach Erfahrungswerten festgestellt werden kann.

**9.** Verfahren nach Anspruch 1 bis 6, **dadurch gekennzeichnet, dass** der Startpunkt **9** eines Fehlers **8** auf einer Oberfläche **2**, dessen Helligkeit sich von der Oberflächenhelligkeit geringfügig unterscheidet, erkannt werden kann, indem für die Berechnung sowohl der maximalen Fehlerhelligkeit $I_{bot}$ bzw. lokalen maximalen Fehlerhelligkeit $I_{bot\_loc}$ als auch der minimalen Oberflächenhelligkeit $I_{top}$ bzw. lokalen minimalen Oberflächenhelligkeit $I_{top\_loc}$ sowie des Helligkeitstrennwertes $I_{sprt}$ anstatt der Konstante $\eta_0$ für die Kantenerkennung ein Helligkeitsfaktor $\eta$ verwendet wird, dessen Größe im Bereich:

$$\eta_0 \leq \eta < 1$$

nach Erfahrungswerten festgestellt werden kann.

10. Verfahren nach Anspruch 1 bis 9, **dadurch gekennzeichnet, dass** der typische Wert für die Standardabweichung $\sigma$ an der Kante eines Fehlers **8** nach Erfahrungswerten festgestellt werden kann.

11. Verfahren nach Anspruch 1 bis 10, **dadurch gekennzeichnet, dass** das Grauwertprofil, das entlang einer Suchlinie **7** gewonnen wird, die quer zu einer typischen Kante **3** verläuft und evtl. mehrmals angelegt werden kann, sowohl aus einer einzelnen Pixelreihe als auch als Mittelwertkurve aus mehreren, z.B. drei, parallel zur Suchlinie **7** liegenden benachbarten Pixelreihen gebildet werden kann.

12. Verfahren nach Anspruch 1 bis 11, **dadurch gekennzeichnet, dass** nur jede einzelne zu untersuchende Kurve aber nicht das Bild selbst geglättet werden soll.

13. Verfahren nach Anspruch 1 bis 12, **dadurch gekennzeichnet, dass** eine morphologische Filterung für die Glättung der zu untersuchenden Kurven verwendet werden soll.

14. Verfahren nach Anspruch 1 bis 13, **dadurch gekennzeichnet, dass** alle Konturpunkte eines Fehlers **8** mit dem gleichen Verfahren wie der Startpunkt **9** ermittelt werden sollen.

**Fig. 1**

**Fig. 2**

**Fig. 3**